Europäisches Patentamt

European Patent Office

Office européen des brevets

(11) Publication number: **0 445 460 B1**

(12) **EUROPEAN PATENT SPECIFICATION**

(45) Date of publication of patent specification: **14.06.95**

(51) Int. Cl.6: **C12N 1/20**, C12P 17/18, C12P 1/06, //(C12N1/20, C12R1:465)

(21) Application number: **90308842.5**

(22) Date of filing: **10.08.90**

(54) **A microorganism for selective production of a specific component of avermectin and a method for selective production thereof.**

(30) Priority: **05.03.90 JP 53411/90**

(43) Date of publication of application: **11.09.91 Bulletin 91/37**

(45) Publication of the grant of the patent: **14.06.95 Bulletin 95/24**

(84) Designated Contracting States: **AT BE CH DE DK ES FR GB IT LI NL**

(56) References cited:
EP-A- 0 276 103
EP-A- 0 313 297
US-A- 4 310 519

(73) Proprietor: **THE KITASATO INSTITUTE**
**5-9-1, Shirokane**
**Minato-ku**
**Tokyo (JP)**

(72) Inventor: **Omura,Satoshi**
**5-12-7,Seta,**
**Setagaya-ku,**
**Tokyo (JP)**
Inventor: **Ikeda,Haruo**
**1-13-627, Kawahara-machi,**
**Saiwai-ku**
**Kawasaki-shi,**
**Kanagawa (JP)**

(74) Representative: **Woods, Geoffrey Corlett et al**
**J.A. KEMP & CO.**
**14 South Square**
**Gray's Inn**
**London WC1R 5LX (GB)**

**Description**

This invention relates to a microorganism belonging to the species Streptomyces avermitilis capable of selective production of a specific component of avermectin.

Avermectins are antibiotics having anthelmintic activity produced by Streptomyces avermitilis. In cultured media of said microorganism, eight components of avermectin, $A_{1a}$, $A_{2a}$, $B_{1a}$, $B_{2a}$, $A_{1b}$, $A_{2b}$, $B_{1b}$ and $B_{2b}$, are produced (US-A 4,310,519). Components "A" and "B" have methoxy or hydroxy substituents at C - 5, respectively. The component group "1" has a double bond at C - 22 and C - 23, and the group "2" has hydrogen at C - 22 and hydroxy at C - 23. An "a" group component has a sec-butyl substituent at C - 25, and a "b" group component has an isopropyl substituent at C - 25. Among these 22, 23-dihydroavermectin $B_1$ (Ivermectin), a hydrogenated product of a $B_1$ component, is used as an anthelmintic.

In the prior art, avermectin has been produced by culturing Streptomyces avermitilis in a medium consisting of an assimilable nitrogen source, carbon source and inorganic salt under aerobic conditions to produce eight components of avermectin $A_{1a}$, $A_{2a}$, $B_{1a}$, $B_{2a}$, $A_{1b}$, $A_{2b}$, $B_{1b}$ and $B_{2b}$ of analogous structure. The product extracted with an organic solvent, a mixture of avermectins, is separated to fractions $A_1$, $A_2$, $B_1$ and $B_2$, then purified to obtain the $B_1$ fraction, which is a mixture of $B_{1a}$ and $B_{1b}$, and thereafter the $B_1$ fraction is hydrogenated to manufacture 22, 23-dihydroavermectin $B_1$.

The prior art has a number of disadvantages. The eight components have to be produced as a mixture. Furthermore an industrial scale separation of "a" and "b" components is quite difficult. Thus an efficient production of the $B_{1a}$ component with good yield and low cost has strongly been requested.

We have found that avermectin $B_{1a}$ and $B_{2a}$ components can effectively be obtained by using a microorganism strain having certain properties.

Thus the present invention provides a microorganism of the species Streptomyces avermitilis having the following properties:

- selective specific accumulation of avermectin component "a",
- effective incorporation of isoleucine or the keto acid thereof (3-methyl-2-oxovaleric acid) into an avermectin molecule, and
- markedly suppressed incorporation of valine or the keto acid thereof (2-oxoisovaleric acid) into an avermectin molecule.

The present invention also provides a process for selective production of a specific component of avermectin which comprises culturing a microorganism as defined above, allowing avermectin $B_{1a}$ to accumulate in the cultured mass, and isolating avermectin $B_{1a}$ therefrom.

The present invention further provides a process for selective production of a specific component of avermectin which comprises culturing a microorganism as defined above, allowing avermectin $B_{1a}$ and $B_{2a}$ to accumulate in the cultured mass, and isolating avermectin $B_{1a}$ and $B_{2a}$ therefrom.

The present invention further provides a process for preparing a microorganism as defined above which comprises mutating a microorganism of the species Streptomyces avermitilis and selecting microorganisms having the properties defined above.

A microorganism deficient in avermectin B O-methyltrans ferase activity is preferred. Separation of avermectin $B_{1a}$ (having a double bond between C-22 and C-23) and $B_{2a}$ (having OH at C-23) components can easily be made by chromatography.

Figure 1A shows the HPLC pattern of a cultured material of Streptomyces avermitilis ATCC 31271;
Figure 1B shows the HPLC pattern of a cultured material of Streptomyces avermitilis K 2033;
Figure 2 shows the HPLC pattern of a cultured material of Streptomyces avermitilis K 2038.
Figure 3 shows the HPLC pattern purified product of cultured Streptomyces avermitilis K 2038.

No report has been known on the effective accumulation of a specific component of avermectin by a fermentation method without feeding additives to a medium during culture using a Streptomyces avermitilis which is deficient in avermectin B O-methyltransferase activity.

Microorganisms having the properties of specific accumulation of avermectin component "a", an effective incorporation of isoleucine or the keto acid thereof into an avermectin molecule, and markedly suppressed incorporation of valine or the keto acid thereof into an avermectin molecule, preferably also deficient in avermectin B O-methyltransferase activity, can be used in the present invention whether obtained from natural sources or being mutants having auxotrophic nature or drug resistance. The present invention also includes microorganisms or a mutant having the properties described in the present specification, which are improved by recombinant DNA techniques, transformation or transduction.

Preferred microorganisms used in the present invention are Streptomyces avermitilis K 2038 which is derived from Streptomyces avermitilis ATCC 31271 and which is a mutant wherein protoplasts of a mutant K 2033 having the properties of specific accumulation of avermectin component "a",an effective incorporation of isoleucine or the keto acid thereof into an avermectin molecule, and markedly suppressed incorporation of valine or the keto acid thereof into an avermectin molecule, and a mutant K 2034 which is deficient in avermectin B O-methyl-transferase activity, are fused. Namely strain K 2038 is a mutant in which deficiency in avermectin B O-methyl-transferase activity is introduced into strain K 2033. These mutant strains, Streptomyces avermitilis K 2033, K 2034 and K 2038, have been deposited in the Fermentation Research Institute, Agency of Industrial Science and Technology, M.I.T.I. Japan, according to Budapest Treaty as FERM BP-2773 FERM BP-2774 and FERM BP-2775, respectively.

Induction of mutation can easily be performed by conventional mutation techniques. Preferably, the original strain is treated by ultraviolet irradiation or with a mutagen such as N-methyl-N'-nitro-N-nitrosoguanidine or ethyl methanesulfonic acid, and thereafter treated colonies are cultured in a medium, in which labeled isoleucine or the keto acid thereof (3-methyl-2-oxo-valeric acid) and labeled valine or the keto acid thereof (2-oxo-isovaleric acid) are added and the culture is further continued for several hours. The avermectin fraction is isolated from cultured mycelia and the radioactivity of avermectin is measured. Thus mutants having the properties of an effective incorporation of isoleucine or the keto acid thereof into an avermectin molecule, and significantly suppressed incorporation of valine or the keto acid thereof into an avermectin molecule are selected.

A mutant deficient in avermectin B O-transferase activity can be obtained by treatment by a mutagen as above, and the thus obtained colonies are cultured in an avermectin production medium. Cultured mycelia are extracted with an organic solvent and the extract is separated by silica-gel thin layer chromatography. The mutant which can merely produce avermectin B can then be selected.

Introduction of the deficiency in avermectin B O-methyltransferase activity by a protoplast fusion technique can be performed by fusing a protoplast prepared from a mutant having the properties of specific accumulation of avermectin component "a", an effective incorporation of isoleucine or the keto acid thereof into an avermectin molecule, and markedly suppressed incorporation of valine or the keto acid thereof into an avermectin molecule, with a protoplast prepared from a mutant having deficiency in avermectin B O-methyltransferase activity, using polyethylene glycol. The fused protoplast is then regenerated to mycelia in a suitable regeneration medium. Regenerated colonies having the properties of accumulating avermectin component "a" and deficiency in avermectin B O-methyl-transferase activity are selected.

In a production of avermectin $B_{1a}$ and $B_{2a}$ , the mutant strain having the properties of specific accumulation of avermectin component "a", an effective incorporation of isoleucine or the keto acid thereof into an avermectin molecule, and markedly suppressed incorporation of valine or the keto acid thereof into an avermectin molecule, and having a deficiency in avermectin B O-methyltransferase activity, are cultured in a medium. This medium is a conventional medium which contains a carbon source, nitrogen source and inorganic salts. Examples of a carbon source are glucose, glycerin, sucrose, dextrin, starch or molasses. Examples of a nitrogen source are caseine, caseine hydrolysate, yeast extract, autolysed yeast, yeast hydrolysate, dry yeast, soy bean powder, soy bean digestible, corn steep liquor, distillar's soluble, cotton seed powder or meat extract. Examples of inorganic salts are phosphate, sulfate, nitrate, chloride or carbonate of sodium, potassium, magnesium, ammonium, calcium, manganese, zinc, iron or cobalt, and conventional salts which can generate these ions. Cultivation can, for example, be carried out under aerobic conditions. Fermentation can, for example, be carried out by controlling the medium to pH 5 - 9, at 25 - 35°C, for 120 - 192 hours shaking the culture under aerobic conditions. If production of avermectin $B_{1a}$ is desired, an avermectin producing strain which can accumulate avermectin $B_{1a}$ should naturally be selected.

Avermectin $B_{1a}$ and $B_{2a}$ can be isolated from the cultured mass by a conventional isolation method for antibiotics. For example, the filtered mycelia may be extracted from a composition containing avermectins with an organic solvent such as acetone or methanol, which is concentrated after filtration. The concentrate is further extracted with an organic solvent such as methylene chloride. The organic layer is concentrated in vacuo to obtain avermectin $B_{1a}$ and $B_{2a}$. Avermectin $B_{1a}$ can be separated by treating the concentrate with column chromatography using an ion-exchanger, silica-gel, reverse phase silica-gel or Sephadex, or by a counter current distribution method. For example, a mixture containing avermectin $B_{1a}$ is treated by preparative HPLC (reverse phase silica-gel, ODS) with a migration phase of 80% v/v methanol/water to elute avermectin $B_{1a}$.The extract is concentrated in vacuo and recrystallized from methanol to obtain avermectin $B_{1a}$ in a pure form.

The following Examples further illustrate the present invention.

Isolation of a mutant having the properties of specific accumulation of avermectin component "a", an effective incorporation of isoleucine or the keto acid thereof into an avermectin molecule, and markedly suppressed incorporation of valine or the keto acid thereof into an avermectin molecule:

Example 1

Spores of Streptomyces avermitilis ATCC 31271 treated by a conventional method with an N-methyl-N'-nitro-N-nitrosoguanidine (1 mg/ml, pH 9.0 at 30°C for 60 min.) treatment were diluted with sterilized water for approximately 200 colonies per plate, spread on a YMS plate and cultured at 30 °C for 5 days. The colonies were picked up and inoculated patchwise onto a YMS plate with 1 cm$^2$ squares, which was set up as a master plate. Each colony on the master plate was inoculated into a 100 ml Erlenmeyer flask containing 10 ml of culture medium, and cultured at 28°C at 210 rpm, amplitude 2.5 cm for 96 hours. [U-$^{14}$C ] -L-isoleucine (50,000 cpm) and [3,4-$^3$H] -L-valine (100,000 cpm) were added thereto and further cultured for 6 hours. Cultured mycelia were collected and extracted with 3 ml acetone. The acetone extract was dried in vacuo. Crude extract dissolved in a small amount of methanol was spotted on a silica-gel thin layer plate (Merck Kiesel gel 60F$_{254}$) and developed with 15% v/v isopropanol/hexane. After checking for an avermectin spot with UV irradiation at 254 nm, the part thereof was cut and put into a 15 ml scintillation vial. 0.5 ml methanol was added and the vial shaken at room temperature for 10 minutes to extract avermectin from the silica-gel. 5 ml scintillator (10 g 2,5 -diphenyloxazol, 0.2 g p-bis(O-methylstyryl) benzene and 1 ℓ xylene) was added therein and the radioactivity of each sample was measured by a liquid scintillation spectrometer. Streptomyces avermitilis ATCC 31271 was used as a control.

Example 2

A corresponding mutant having the properties of an effective incorporation of [U-$^{14}$C ] -L-isoleucine into an avermectin molecule, and markedly suppressed incorporation of [3,4-$^3$H] -L-valine into an avermectin molecule, was collected from the master plate and cultured as in Example 1. An equal amount as in Example 1 of [U-$^{14}$C ] -L-isoleucine and [3,4-$^3$H] -L-valine, or [U-$^{14}$C ] -3-methyl-2-oxovaleric acid and [3,4-$^3$H] -2-oxoisovaleric acid were added and cultured for 6 hours. The avermectin fraction was isolated as in Example 1 and the radioactivity was measured. The result is shown in Table 1. Labeled compound was incorporated into the avermectin molecule by an original strain Streptomyces avermitilis ATCC 31271. On the other hand, mutant strain K 2033 incorporated [U-$^{14}$C ] - L-isoleucine or the keto acid thereof (3-methyl-2-oxovaleric acid) effectively into an avermectin molecule, but incorporation of [3,4-$^3$H] -L-valine or the keto acid thereof (2-oxoisovaleric acid) into an avermectin molecule was markedly decreased.

Example 3

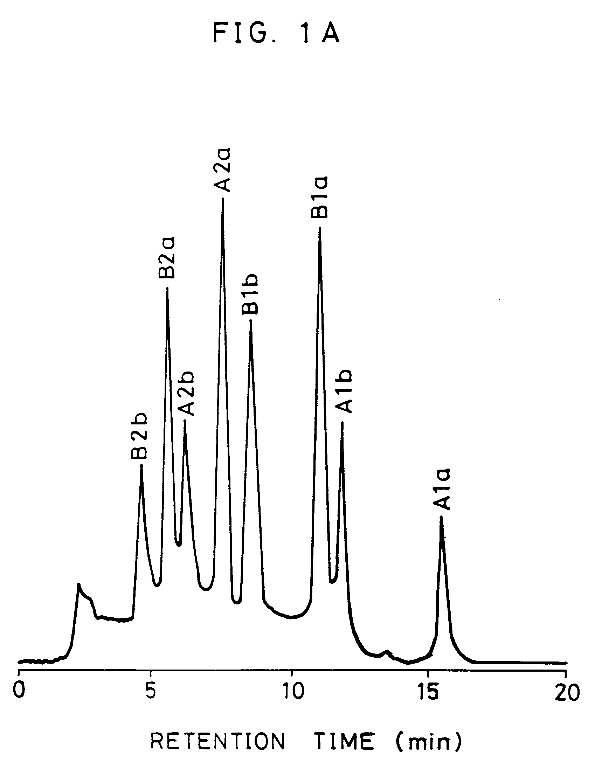

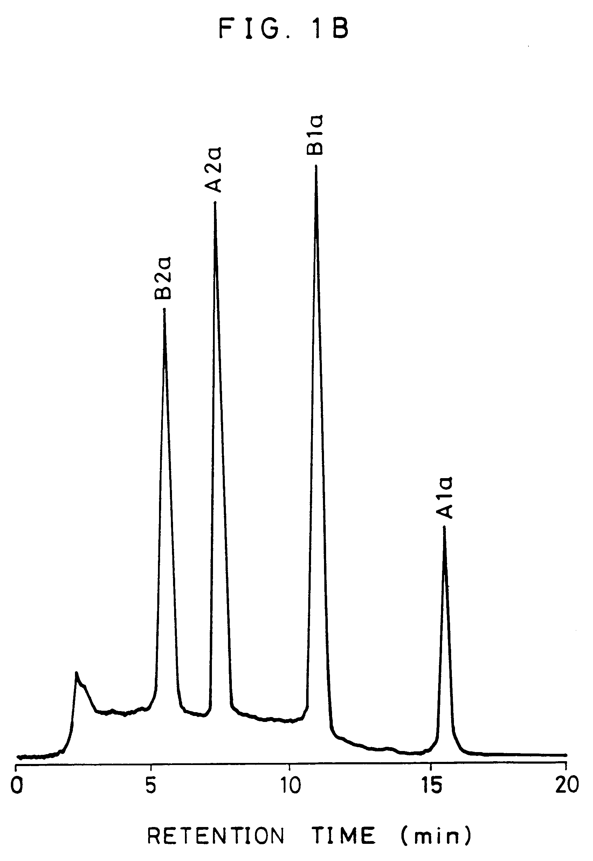

Spore suspensions of the original strain Streptomyces avermitilis ATCC 31271 and strain K 2033 obtained in Example 2 were inoculated into a 100 ml Erlenmeyer flask containing 10 ml production medium, and cultured at 28 °C, 210 rpm, amplitude 2.5 cm, for 168 hours. Cultured mycelia were mixed with 10 ml methanol and the avermectin was extracted. After removing the mycelial residue by centrifugation, avermectin in the extract was analysed by HPLC (ODS 3 $\mu$m, column size 6 $\phi$ × 75 mm, speed 1 ml/min., mobility phase 80% v/v methanol/water, detection 246 nm). The result is shown in Figs. 1A and 1B. Original strain Streptomyces avermitilis ATCC 31271 produced 8 components of avermectin (A$_{1a}$ , A$_{1b}$ , A$_{2a}$ , A$_{2b}$ , B$_{1a}$ , B$_{1b}$ , B$_{2a}$ and B$_{2b}$ ) and mutant strain K 2033 produced 4 components of avermectin containing the main effective component B$_{1a}$ (A$_{1a}$ , A$_{2a}$ , B$_{1a}$ and B$_{2a}$ ).

Mutant strain deficient in avermectin B O-methyl transferase

Example 4

Spores of Streptomyces avermitilis ATCC 31271 treated by a conventional method with a N-methyl-N'-nitro-N-nitrosoguanidine (1 mg/ml, pH 9.0 at 30 °C for 60 min.) treatment were diluted with sterilized water for approximately 200 colonies per plate, spread on a YMS plate and cultured at 30 °C for 5 days. The colonies were picked up and inoculated patchwise onto YMS plate with 1 cm$^2$ squares, which was set up as a master plate. The master plate was replicated on the production medium 3 and incubated at 30°C for 8 days. Each patch-like shape colony on the production medium 3 was cut-out together with agar medium, inserted into a plastic tube, 0.5 ml acetone was added therein and allowed to stand at room temperature for 15 minutes to extract the cultured product. After removing mycelia and agar strips, the acetone extract was dried in vacuo. The extract was dissolved in 25 $\mu$ l acetone, and 5 $\mu$ l thereof was spotted on a silica-gel thin layer plate (Merck, Kiesel gel $60F_{254}$) then developed with 15% v/v isopropanol/hexane. A mutant strain K 2034 which produced only avermectin B component was collected by means of UV irradiation at 254 nm.

Introduction of avermectin B O-methyltransferase deficient property into strain K 2033 by protoplast fusion:

Example 5

A spore suspension of strain K 2033 obtained in Example 2 and avermectin B O-methyltransferase activity deficient strain K 2034 obtained by mutation of original strain ATCC 31271 were inoculated into a 500 ml Erlenmeyer flask containing YEME medium (50 ml) including 30% w/v sucrose, 5mM $MgCl_2$ and 0.5% w/v glycine, and cultured at 28°C, 180 rpm for 68 hours. Mycelia obtained by centrifugation of the cultured liquid at 3000 rpm for 10 min. was suspended in P10 medium (10 ml) and re-centrifuged to collect mycelia. Washed mycelia were suspended in P10 medium (10 ml) containing egg white lysozyme (1 mg/ml) sterilized by filtration and gently shaken at 37°C for 60 minutes to form a protoplast. A sample containing the protoplast was filtered through a cotton filter to remove undigested mycelia. The protoplast was sedimented by centrifugation at 3000 rpm for 10 minutes. The protoplast was gently suspended by adding P 20 medium (5 ml), and thereafter again the protoplast was collected by centrifugation. After resuspension in protoplastin P 20 medium (2 ml), a part of the suspendion was diluted and dropped onto a hemocytometer. The number of protoplasts was then calculated with a phase-contrast microscope ($\times$ 400). The protoplasts of strain K 2033 and K 2034, each $5 \times 10^8$, respectively, were transferred into a small test tube and mixed completely. The whole volume thereof was controlled to below 50 $\mu$ l. A solution (0.5 ml) of 50% w/v polyethylene glycol #1000 (1g of polyethylene glycol #1000 dissolved in 1ml of P 20 medium was filter-sterilized through 0.45 $\mu$ m filter) was added therein and rapidly mixed to fuse the protoplasts. After allowing to stand at room temperature for 1 minute, P 20 medium (0.5 ml) was added and mixed to dilute the polyethylene glycol. A fusant was diluted with P 20 medium at $10^{-2}$ and $10^{-3}$, and 0.1 ml/plate of the fusant and 2.5 ml of soft agar RM14 were spread on RM14 medium. The plate was incubated at 30 °C for 10 days to regenerate the mycelia. The mycelia were separated from the plate surface and homogenated by a homogenizer. The mycelia were diluted with sterile water, spread on a YMS plate and incubated at 30°C for 5 days. Matured spores were scratched up, diluted with sterile water up to 200 colonies per plate, spread on a YMS plate then incubated at 30°C for 5 days. 80% of the budding colonies were of K 2033 strain type (dark brown, abundant spores) and the others were of K 2034 strain type (pale brown, few spores).

Example 6

Colonies appearing of an analogous type to K 2033 strain obtained by protoplast fusion were spread patchwise and incubated at 30 °C for 8 days. Patches of each colony were cut-out, inserted into plastic tubes, acetone (0.5 ml) was added thereto and allowed to stand at room temperature for 15 minutes for extracting the product. Mycelia and agar strips were removed and the extract was concentrated in vacuo. The thus obtained crude extract was dissolved in acetone (25 $\mu$ l) and the solution (5 $\mu$ l) was spotted on a silica-gel thin layer plate, then developed with 85% v/v hexane/isopropanol. Avermectin thus produced was checked by UV at 254 nm, then strains which produced avermectin B component having a deficiency in avermectin B O-methyltransferase activity were selected. These extracts were further spotted on a reversed phase silica-gel thin layer plate (Whatman KC18F), developed with 70% v/v acetonitrile/water, then strains merely producing avermectin "a" component were selected by checking with UV at 254 nm.

Example 7

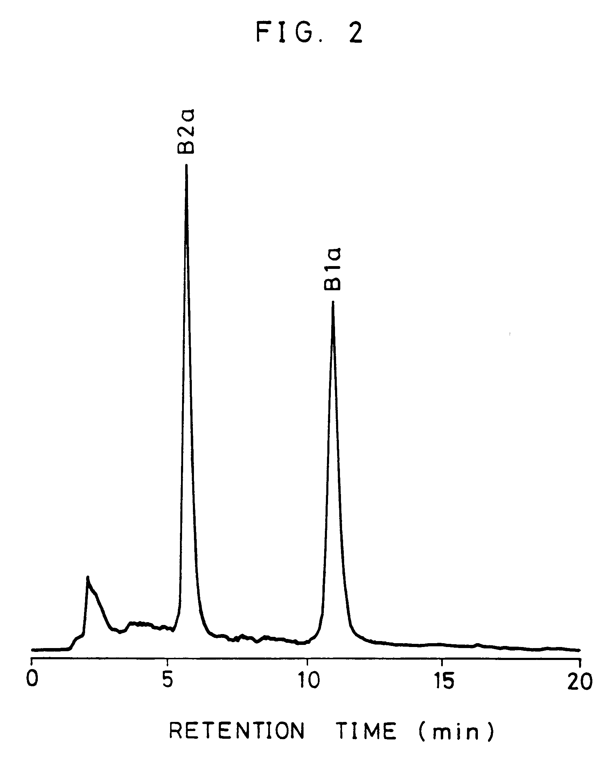

Spore suspension of a variant (K 2038 strain) which was obtained by protoplast fusion and was merely producing avermectin "a" component in the B component, was inoculated in a 50 ml test tube containing 10 ml seed medium and shake cultured at 30 °C for 48 hours. A 0.2 ml sample thereof was inoculated into a 100 ml Erlenmeyer flask containing 10 ml production medium 2 and cultured at 28°C at 210 rpm, 2.5 cm amplitute for 168 hours. Cultured product was extracted in the same way as in Example 3, then the extract was analysed. The result is shown in Fig. 2. A strain K 2038 obtained by protoplast fusion produced the active principles components $B_{1a}$ and $B_{2a}$ in the 8 components of avermectins. Furthermore the amount

EP 0 445 460 B1

avermectin $B_{1a}$ accumulated was significantly increased.

Example 8

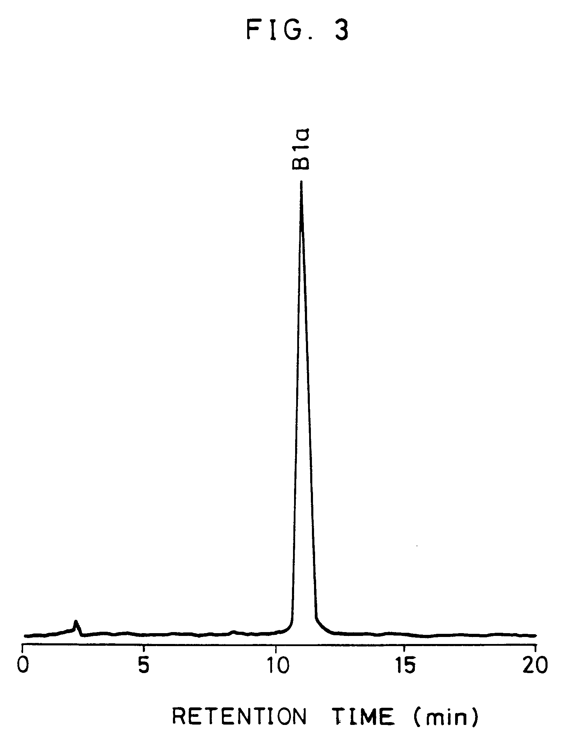

Mycelia were separated from cultured mass (approx. 250 ml) of Streptomyces avermitilis K 2038 cultured in 30 Erlenmeyere flasks. After washing the mycelia with deionized water (150 ml), methanol (100 ml) was added thereto and stirred at room temperature for 30 minutes for extraction. Mycelial extract was filtered through Celite to remove mycelial residue, and the filtrate was concentrated to approximately 10 ml in vacuo. Deionized water (10 ml) and further methylene chloride (20 ml) were added to the concentrate for extraction. The methylene chloride layer was separated, and thereafter the water layer was extracted with methylene chloride (20 ml). The combined methylene chloride extract was dried in vacuo and the residue was dissolved in ethyl acetate (50 ml) which was dehydrated by adding anhydrous sodium sulfate (2 g). An ethyl acetate solution was passed through a silica-gel (10 g) column and then eluted with ethyl acetate (50 ml). The eluate was collected and concentrated in vacuo to obtain a viscous oily product (0.8 g). The oily substance (0.8 g) dissolved in a small amount of methylene chloride was passed through a column of silica-gel (Merck 100 - 200 mesh) equilibrated with methylene chloride. After washing with methylene chloride, elution was carried out with 5% v/v isopropanol/methylene chloride. Fractions containing avermectin $B_{1a}$ were collected then passed through a column of active carbon (2 g). The column was further washed with methylene chloride (10 ml). The thus obtained eluate was dried in vacuo, and added thereto was a small amount of isopropyl ether to dissolve the material. Thereafter hexane was added dropwise under cooling to obtain a white precipitate. The precipitate was collected by filtration, and dried in vacuo to obtain a white powder (10 mg). According to analysis by HPLC the white powder contained over 90% of avermectin $B_{1a}$. (refer to Fig. 3)

The physico-chemical properties of the thus obtained white powder are identical with those of avermectin $B_{1a}$ reported in J. Am. Chem. Soc., 103: 4216 - 4221 (1981).

The compositions of the media used in the foregoing Examples are illustrated hereinbelow:

YMS medium

| malt extract (Difco) | 10 g |
|---|---|
| yeast extract (Difco) | 4 g |
| soluble starch (Difco) | 4 g |
| agar | 20 g |
| distilled water | 1 ℓ |

Adjusted to pH 7.2 with 2N KOH and sterilized at 121 °C for 15 minutes. After sterilization, magnesium chloride and calcium nitrate were added up to 10 mM and 8 mM, respectively.

Production medium 1

| glucose | 30 g |
|---|---|
| NaCℓ | 2.0 g |
| $KH_2PO_4$ | 0.05 g |
| $FeSO_4 \cdot 7H_2O$ | 0.05 g |
| $ZnSO_4 \cdot 7H_2O$ | 0.05 g |
| $MnSO_4 \cdot 4H_2O$ | 0.05 g |
| $MgSO_4 \cdot 7H_2O$ | 0.1 g |
| $(NH_4)_2SO_4$ | 1.5 g |
| $CaCO_3$ | 5.0 g |
| distilled water | 1 ℓ |

Adjusted to pH 7.2 with 2N KOH, then sterilized at 121 °C for 15 min.

6

Production medium 2

| glucose | 45 g |
|---|---|
| peptonized milk (Oxoid) | 24 g |
| autolyzed yeast (Difco) | 2.5 g |
| polypropylene glycol #2000 | 2.5 m $\ell$ |
| distilled water | 1 $\ell$ |

Adjusted to pH 7.2 with 2N KOH, then sterilized at 121 °C for 15 minutes.

Production medium 3

| glucose | 45 g |
|---|---|
| peptonized milk (Oxoid) | 24 g |
| autolyzed yeast (Difco) | 2.5 g |
| agar | 20 g |
| distilled water | 1 $\ell$ |

Adjusted to pH 7.2 with 2N KOH, then sterilized at 121 °C for 15 minutes.

YEME medium

| yeast extract (Difco) | 3 g |
|---|---|
| malt extract (Difco) | 3 g |
| peptone (Difco) | 5 g |
| glucose | 10 g |
| distilled water | 1 $\ell$ |

Sterilized at 121 °C for 15 minutes.

Trace element solution

| $FeC\ell_3 \cdot 6H_2O$ | 200 mg |
|---|---|
| $ZnC\ell_2$ | 40 mga |
| $CuC\ell_2 \cdot 2H_2O$ | 10 mg |
| $MnC\ell_2 \cdot 4H_2O$ | 10 mg |
| $Na_2B_4O_7 \cdot 10H_2O$ | 10 mg |
| $(NH_4)_6Mo_7O_{24} \cdot 4H_2O$ | 10 mg |
| distilled water | 1 $\ell$ |

P 10 medium

| sucrose | 103 g |
|---|---|
| $K_2SO_4$ | 0.25 g |
| $MgCl_2 \cdot 6H_2O$ | 2.03 g |
| trace element solution | 2.0 m $\ell$ |
| distilled water | 800 m $\ell$ |

After sterilization at 121 °C for 15 minutes the following mixture is added.

| 0.5 % $KH_2PO_4$ | 10 m $\ell$ |
|---|---|
| 3.68 % $CaCl_2 \cdot 2H_2O$ | 100 m $\ell$ |
| 0.25 M MES buffer solution pH 6.5 | 100 m $\ell$ |

P 20 medium

| sucrose | 205 g |
|---|---|
| $K_2SO_4$ | 0.25 g |
| $MgCl_2 \cdot 6H_2O$ | 2.03 g |
| trace element solution | 2.0 m $\ell$ |
| distilled water | 800 m $\ell$ |

After sterilization at 121 °C for 15 minutes the following mixture is added.

| 0.5 % $KH_2PO_4$ | 10 m $\ell$ |
|---|---|
| 3.68 % $CaCl_2 \cdot 2H_2O$ | 100 m $\ell$ |
| 0.25 M MES buffer solution pH 6.5 | 100 m $\ell$ |

RM 14 medium

| sucrose | 205 g |
|---|---|
| $K_2SO_4$ | 0.25 g |
| $MgCl_2 \cdot 6H_2O$ | 10.12 g |
| glucose | 10.0 g |
| casamino acid | 0.1 g |
| L-proline | 3.0 g |
| yeast extract (Difco) | 2.0 g |
| trace element solution | 2.0 m $\ell$ |
| oat meal agar (Difco) | 3.0 g |
| agar | 20 g |
| distilled water | 870 m $\ell$ |

After sterilization at 121 °C for 15 minutes the following mixture is added.

| | |
|---|---|
| 0.05 % KH$_2$SO$_4$ | 10 m ℓ |
| 3.68 % CaCℓ$_2$ • H$_2$SO$_4$ | 80 m ℓ |
| 0.25 M MES buffer solution pH 6.5 | 40 m ℓ |

<u>Soft agar RM 14 medium</u>

| | |
|---|---|
| sucrose | 205 g |
| K$_2$SO$_4$ | 0.25 g |
| MgCℓ$_2$ • 6H$_2$O | 10.12 g |
| glucose | 10.0 g |
| casamino acid (Difco) | 0.1 g |
| L-proline | 3.0 g |
| yeast extract (Difco) | 2.0 g |
| trace element solution | 2.0 m ℓ |
| oat meal agar (Difco) | 3.0 g |
| agar | 5.0 g |
| distilled water | 870 m ℓ |

After sterilization at 121 ° C for 15 minutes the following mixture is added.

| | |
|---|---|
| 0.5 % KH$_2$PO$_4$ | 10 m ℓ |
| 3.68 % CaCℓ$_2$ • 6H$_2$O | 80 m ℓ |
| 0.25 M MES buffer solution pH 6.5 | 40 m ℓ |

Table 1

| strain | Incorporation of labeled compound into avermectin ( cpm ) | | | |
| --- | --- | --- | --- | --- |
| | Additive | | | |
| | $(U-{}^{14}C)-L-$ isoleucine | $(3, 4-{}^3H)-$ L − valine | $(U-{}^{14}C)-3-methyl$ $-2-$ oxovaleric acid | $(3, 4-{}^3H)-2$ − oxovaleric acid |
| ATCC31271 | 398 | 423 | 655 | 575 |
| K2033 | 512 | 22 | 927 | 25 |

EP 0 445 460 B1

EP 0 445 460 B1

**Claims**

**Claims for the following Contracting States : GB, FR, DE, NL, CH, LI, DK, AT, IT, BE**

1. A microorganism of the species Streptomyces avermitilis avermitilis having the following properties:
   - selective specific accumulation of avermectin component "a",
   - effective incorporation of isoleucine or the keto acid thereof (3-methyl-2-oxovaleric acid) into an avermectin molecule, and
   - markedly suppressed incorporation of valine or the keto acid thereof (2-oxoisovaleric acid) into an avermectin molecule.

2. A microorganism according to claim 1 which is a mutant of Streptomyces avermitilis ATCC 31271, said mutant having the properties defined in claim 1.

3. A microorganism according to claim 2 which is Streptomyces avermitilis K 2033 FERM BP-2773 or a mutant thereof, said mutant having the properties defined in claim 1.

4. A microorganism according to claim 2 which is Streptomyces avermitilis K 2038 FERM BP-2775 or a mutant thereof, said mutant having the properties defined in claim 1.

5. A microorganism according to any one of the preceding claims which is deficient in avermectin B O-methyltransferase activity.

6. A process for preparing a microorganism as claimed in any one of the preceding claims which comprises mutating a microorganism of the species Streptomyces avermitilis and selecting microorganisms having the properties defined in claim 1.

7. A process for selective production of a specific component of avermectin which comprises culturing a microorganism as claimed in any one of claims 1 to 5, allowing avermectin $B_{1a}$ to accumulate in the cultured mass, and isolating avermectin $B_{1a}$ therefrom.

8. A process for selective production of a specific component of avermectin which comprises culturing a microorganism as claimed in any one of claims 1 to 5, allowing avermectin $B_{1a}$ and $B_{2a}$ to accumulate in the cultured mass, and isolating avermectin $B_{1a}$ and $B_{2a}$ therefrom.

**Claims for the following Contracting State : ES**

1. A process for preparing a microorganism of the species Streptomyces avermitilis having the following properties:
   - selective specific accumulation of avermectin component "a",
   - effective incorporation of isoleucine or the keto acid thereof (3-methyl-2-oxovaleric acid) into an avermectin molecule, and
   - markedly suppressed incorporation of valine or the keto acid thereof (2-oxoisovaleric acid) into an avermectin molecule;
   said process comprising mutating a microorganism of the species Streptomyces avermitilis and selecting microorganisms having the properties defined above.

2. A process according to claim 1 wherein the starting microorganism is Streptomyces avermitilis ATCC 31271.

3. A process according to claim 2 wherein the microorganism obtained is Streptomyces avermitilis K 2033 FERM BP-2773 or a mutant thereof, said mutant having the properties defined in claim 1.

4. A process according to claim 2 wherein the microorganism obtained is Streptomyces avermitilis K 2038 FERM BP-2775 or a mutant thereof, said mutant having the properties defined in claim 1.

5. A process according to any one of the preceding claims wherein the microorganism obtained is deficient in avermectin B O-methyltransferase activity.

11

6.  A process for selective production of a specific component of avermectin which comprises culturing a microorganism of the species <u>Streptomyces</u> <u>avermitilis</u> as defined in any one of the preceding claims, said microorganism having the properties defined in claim 1, allowing avermectin $B_{1a}$, or $B_{1a}$ and $B_{2a}$,to accumulate in the cultured mass, and isolating avermectin $B_{1a}$, or $B_{1a}$ and $B_{2a}$, therefrom.

**Patentansprüche**
**Patentansprüche für folgende Vertragsstaaten : GB, FR, DE, NL, CH, LI, DK, AT, IT, BE**

1.  Mikroorganismus der Species Streptomyces avermitilis mit den folgenden Eigenschaften:
    - selektive spezifische Anreicherung der Avermectin-Komponente "a",
    - wirksamer Einbau von Isoleucin oder seiner Ketosäure (3-Methyl-2-oxovaleriansäure) in ein Avermectinmolekül, und
    - ausgeprägt unterdrückter Einbau von Valin oder seiner Ketosäure (2-Oxoisovaleriansäure) in ein Avermectinmolekül.

2.  Mikroorganismus nach Anspruch 1, dadurch gekennzeichnet, daß er eine Mutante von Streptomyces avermitilis ATCC 31271 ist, wobei diese Mutante die im Anspruch 1 definierten Eigenschaften besitzt.

3.  Mikroorganismus nach Anspruch 2, dadurch gekennzeichnet, daß er Streptomyces avermitilis K 2033 FERM BP-2773 oder eine Mutante davon ist, wobei diese Mutante die im Anspruch 1 definierten Eigenschaften besitzt.

4.  Mikroorganismus nach Anspruch 2, dadurch gekennzeichnet, daß er Streptomyces avermitilis K 2038 FERM BP 2775 oder eine Mutante davon ist, wobei diese Mutante die in Anspruch 1 definierten Eigenschaften besitzt.

5.  Mikroorganismus nach einem der vorhergehenden Ansprüche, dadurch gekennzeichnet, daß er einen Mangel an Avermectin B O-Methyltransferase-Aktivität aufweist.

6.  Verfahren zur Herstellung eines Mikroorganismus nach einem der vorhergehenden Ansprüche, dadurch gekennzeichnet, daß man einen Mikroorganismus der Species Streptomyces avermitilis mutiert und Mikroorganismen auswählt, die die im Anspruch 1 definierten Eigenschaften besitzen.

7.  Verfahren zur selektiven Herstellung einer spezifischen Komponente von Avermectin, dadurch gekennzeichnet, daß man einen in einem der Ansprüche 1 bis 5 beanspruchten Mikroorganismus kultiviert, Avermectin $B_{1a}$ in der kultivierten Masse anreichern läßt, und das Avermectin $B_{1a}$ daraus isoliert.

8.  Verfahren zur selektiven Herstellung einer spezifischen Komponente von Avermectin, dadurch gekennzeichnet, daß man einen in einem der Ansprüche 1 bis 5 beanspruchten Mikroorganismus kultiviert, Avermectin $B_{1a}$ und $B_{2a}$ in der kultivierten Masse anreichen läßt, und Avermectin $B_{1a}$ und $B_{2a}$ daraus isoliert.

**Patentansprüche für folgenden Vertragsstaat : ES**

1.  Verfahren zur Herstellung eines Mikroorganismus der Species Streptomyces avermitilis mit den folgenden Eigenschaften:
    - selektive spezifische Anreicherung der Avermectin-Komponente "a",
    - wirksamer Einbau von Isoleucin oder seiner Ketosäure (3-Methyl-2-oxovaleriansäure) in ein Avermectinmolekül, und
    - ausgeprägt unterdrückter Einbau von Valin oder seiner Ketosäure (2-Oxoisovaleriansäure) in ein Avermectinmolekül; dadurch gekennzeichnet, daß man Mikroorganismus des Species Streptomyces avermitilis mutiert und Mikroorganismen mit den vorstehend definierten Eigenschaften auswählt.

2.  Verfahren nach Anspruch 1, dadurch gekennzeichnet, daß der Ausgangsmikroorganismus Streptomyces avermitilis ATCC 31271 ist.

3. Verfahren nach Anspruch 2, dadurch gekennzeichnet, daß der erhaltene Mikroorganismus Streptomyces avermitilis K 2038 FERM BP-2773 oder eine Mutante davon ist, wobei diese Mutante die im Anspruch 1 definierten Eigenschaften besitzt.

4. Verfahren nach Anspruch 2, dadurch gekennzeichnet, daß der erhaltene Mikroorganismus Streptomyces avermitilis K 2033 FERM BP 2775 oder eine Mutante davon ist, wobei diese Mutante die in Anspruch 1 definierten Eigenschaften besitzt.

5. Verfahren nach einem der vorhergehenden Ansprüche, dadurch gekennzeichnet, daß der erhaltene Mikroorganismus einen Mangel an Avermectin B O-Methyltransferase-Aktivität aufweist.

6. Verfahren zur selektiven Herstellung einer spezifischen Komponente von Avermectin, dadurch gekennzeichnet, daß man einen in einem der vorhergehenden Ansprüche definierten Mikroorganismus der Species Streptomyces avermitilis kultiviert, wobei diese Mikroorganismus die im Anspruch 1 definierten Eigenschaften besitzt, Avermectin $B_{1a}$, oder $B_{1a}$ und $B_{2a}$, in der kultivierten Masse anreichen läßt, und Avermectin $B_{1a}$, oder $B_{1a}$ und $B_{2a}$, daraus isoliert.

**Revendications**
**Revendications pour les Etats contractants suivants : GB, FR, DE, NL, CH, LI, DK, AT, IT, BE**

1. Micro-organisme de l'espèce Streptomyces avermitilis présentant les propriétés suivantes :
   - accumulation spécifique sélective du composant "a" de l'avermectine,
   - incorporation efficace d'isoleucine ou de son céto-acide (acide 2-méthyl-2-oxovalérique) dans une molécule d'avermectine, et
   - incorporation notablement supprimée de valine ou de son céto-acide (acide 2-oxoisovalérique) dans une molécule d'avermectine.

2. Micro-organisme selon la revendication 1, qui est un mutant de Streptomyces avermitilis ATCC 31271, ledit mutant présentant les propriétés définies à la revendication 1.

3. Micro-organisme selon la revendication 2, qui est Streptomyces avermitilis K 2033 FERM BP-2773 ou un mutant de celui-ci, ledit mutant présentant les propriétés définies à la revendication 1.

4. Micro-organisme selon la revendication 2, qui est Streptomyces avermitilis K 2038 FERM BP-2775 ou un mutant de celui-ci, ledit mutant ayant les propriétés définies à la revendication 1.

5. Micro-organisme selon l'une des revendications précédentes qui est dépourvu d'activité de O-méthyl-transférase de l'avermectine B.

6. Procédé pour préparer un micro-organisme selon l'une quelconque des revendications précédentes, qui consiste à muter un micro-organisme de l'espèce Streptomyces avermitilis et à sélectionner les micro-organismes ayant les propriétés définies à la revendication 1.

7. Procédé pour la production sélective d'un composant spécifique de l'avermectine, qui consiste à cultiver un micro-organisme selon l'une quelconque des revendications 1 à 5, à laisser l'avermectine $B_{1a}$ s'accumuler dans la masse cultivée, et à isoler de celle-ci l'avermectine $B_{1a}$.

8. Procédé pour la production sélective d'un composant spécifique de l'avermectine, qui consiste à cultiver un micro-organisme selon l'une quelconque des revendications 1 à 5, à laisser l'avermectine $B_{1a}$ et $B_{2a}$ s'accumuler dans la masse cultivée et à isoler de celle-ci l'avermectine $B_{1a}$ et $B_{2a}$.

**Revendications pour l'Etat contractant suivant : ES**

1. Procédé pour préparer un micro-organisme de l'espèce Streptomyces avermitilis présentant les propriétés suivantes :
   - accumulation spécifique sélective du composant "a" de l'avermectine,
   - incorporation efficace d'isoleucine ou de son céto-acide (acide 3-méthyl-2-oxovalérique) dans une molécule d'avermectine, et

- incorporation notablement supprimée de valine ou de son céto-acide (acide 2-oxoisovalérique) dans une molécule d'avermectine;

ce procédé consistant à muter un micro-organisme de l'espèce Streptomyces avermitilis et à sélectionner les micro-organismes ayant les propriétés définies ci-dessus.

2. Procédé selon la revendication 1, dans lequel le micro-organisme de départ est Streptomyces avermitilis ATCC 31271.

3. Procédé selon la revendication 2, dans lequel le micro-organisme obtenu est Streptomyces avermitilis K 2033 FERM BP-2773 ou un mutant de celui-ci, ledit mutant présentant les propriétés définies à la revendication 1.

4. Procédé selon la revendication 2, dans lequel le micro-organisme obtenu est Streptomyces avermitilis K 2038 FERM BP-2775 ou un mutant de celui-ci, ledit mutant présentant les propriétés définies à la revendication 1.

5. Procédé selon l'une quelconque des revendications précédentes, dans lequel le micro-organisme obtenu est dépourvu d'activité de O-méthyl-transférase de l'avermectine B.

6. Procédé pour la production sélective d'un composant spécifique de l'avermectine qui consiste à cultiver un micro-organisme de l'espèce Streptomyces avermitilis comme défini dans l'une quelconque des revendications précédentes, ledit micro-organisme ayant les propriétés définies à la revendication 1, à laisser l'avermectine $B_{1a}$, ou $B_{1a}$ et $B_{2a}$, s'accumuler dans la masse cultivée, et à isoler de celle-ci l'avermectine $B_{1a}$, ou $B_{1a}$ et $B_{2a}$.

FIG. 1 A

RETENTION TIME (min)

# FIG. 1B

FIG. 2

# FIG. 3

RETENTION TIME (min)